# EUROPÄISCHE PATENTSCHRIFT

(11) **EP 2 679 272 B1**
(45) Veröffentlichungstag und Bekanntmachung des Hinweises auf die Patenterteilung: **17.04.2019**
(21) Anmeldenummer: 13171024.6
(22) Anmeldetag: 07.06.2013
(51) Int. Cl.: A61M 39/06, A61B 17/34

(54) **Trokarvorrichtung**
Trocar device
Dispositif de trocart

(30) Priorität: 26.06.2012 DE 102012210837
(43) Veröffentlichungstag der Anmeldung: 01.01.2014
(73) Patentinhaber: Henke-Sass, Wolf GmbH, 78532 Tuttlingen (DE)
(72) Erfinder: Pauli, Sabine, 78606 Seitingen-Oberflacht (DE); Häckl, Norbert, 88637 Leibertingen (DE); Fuchs, Michael, 78606 Seitingen-Oberflacht (DE)
(74) Vertreter: Patentanwälte Geyer, Fehners & Partner mbB

(56) Entgegenhaltungen:
- EP-A1- 0 517 248
- EP-A1- 2 036 508
- EP-A1- 2 233 089
- WO-A1-98/50093

## Beschreibung

Die vorliegende Erfindung betrifft eine Trokarvorrichtung gemäß dem Oberbegriff des Anspruches 1.

Eine solche Trokarvorrichtung ist z.B. aus der EP 0 517 248 A1 bekannt und wird häufig für einen chirurgischen Eingriff eingesetzt, wobei das Schaftrohr zumindest teilweise innerhalb des zu operierenden Menschen oder Tieres angeordnet ist und das Hauptteil außerhalb angeordnet ist. Über diesen Zugang können dann entsprechende Instrumente durch die Durchgangsöffnung geführt werden, wodurch der gewünschte chirurgische Eingriff ermöglicht ist. Insbesondere kann ein Endoskop durch die Durchgangsöffnung eingeführt werden.

Bei einem solchen Einsatz der Trokarvorrichtung soll verhindert werden, daß Flüssigkeiten aus dem zweiten Bereich (beispielsweise Spülflüssigkeiten oder sonstige Körperflüssigkeiten) in den ersten Bereich gelangen und das Instrument (z.B. das Endoskop) beschädigen. Daher ist eine Dichtungseinheit vorgesehen. Diese war bisher als hohlzylinderförmige Dichtung ausgebildet, die im Hauptteil eingeklebt ist. Damit kann jedoch keine dauerhafte Fixierung der Dichtung erreicht werden, was dann in unerwünschter Weise zu Undichtigkeiten führt.

Ausgehend hiervon ist es daher Aufgabe der Erfindung, die Trokarvorrichtung der eingangs genannten Art so weiterzubilden, daß die beschriebenen Schwierigkeiten möglichst vollständig behoben sind.

Erfindungsgemäß wird die Aufgabe bei einer Trokarvorrichtung der eingangs genannten Art dadurch gelöst, dass das Dichtungselement eine hohlzylinderförmige Wandung aufweist, als Hohlzylinder ausgebildet ist und die Dichtungseinheit einen im Hauptteil befestigten Halter umfaßt, der einen Abschnitt der hohlzylinderförmigen Wandung des Dichtungselementes einklemmt, so daß das Dichtungselement im Halter fixiert ist.

Durch diese Klemmhalterung des Dichtungselementes ist das Dichtungselement sicher und dauerhaft im Halter fixiert. Der Halter ist seinerseits sicher und dauerhaft im Hauptteil befestigt, so daß insgesamt eine dauerhafte Dichtung zwischen den zwei Bereichen der Durchgangsöffnung vorliegt.

Die Dichtungseinheit ist leicht herstellbar, da das Dichtungselement lediglich im Hauptteil eingeklemmt ist. Dadurch kann das Dichtungselement für die Fixierung im Halter frei von Einschnitten sein, die einerseits aufwendig herzustellen wären und andererseits das Dichtungselement selbst schwächen würden. Die Fixierung des Dichtungselementes wird durch das Einklemmen bzw. Zusammendrücken des entsprechenden Wandungsabschnittes im Halter erzielt.

Das elastische Dichtungselement ist als Hohlzylinder ausgebildet, der die hohlzylinderförmige Wandung umfaßt. Unter einem Hohlzylinder wird hier insbesondere verstanden, daß der Innenquerschnitt und/oder der Wandungsquerschnitt des Hohlzylinders in Längsrichtung der hohlzylinderförmigen Wandung gleich bleibt. Das elastische Dichtungselement kann ausschließlich die Form eines Hohlzylinders aufweisen.

Die erfindungsgemäße Trokarvorrichtung kann auch als Führungsvorrichtung für ein Endoskop bezeichnet werden und kann im medizinischen Bereich sowie im nicht medizinischen Bereich eingesetzt werden.

Der Halter kann ein erstes Halteelement aufweisen, das an der Innenseite der Wandung anliegt. Des weiteren kann der Halter ein zweites Halteelement aufweisen, das an der Außenseite der Wandung anliegt. Damit kann in einfacher Art und Weise das gewünschte Einklemmen realisiert werden.

Insbesondere kann das erste Halteelement einen ersten Klemmabschnitt aufweisen, der zum Einklemmen des Wandungsabschnittes radial nach außen vorsteht. Man kann auch sagen, daß der Außendurchmesser des Klemmabschnittes größer ist als der Innendurchmesser der Innenseite der Wandung.

Ferner kann zusätzlich oder alternativ das zweite Halteelement einen zweiten Klemmabschnitt aufweisen, der zum Einklemmen des Wandungsabschnittes radial nach innen vorsteht. Somit weist der zweite Klemmabschnitt einen Innendurchmesser auf, der geringer ist als der Außendurchmesser der hohlzylinderförmigen Wandung.

Zur Befestigung des Halters im Hauptteil kann der Halter ein Außengewinde aufweisen. In diesem Fall wird er in ein entsprechendes Innengewinde im Hauptteil eingeschraubt. Das Außengewinde kann z.B. am zweiten Halteelement ausgebildet sein.

Der Halter kann mit dem Dichtungselement verklebt sein. Ferner kann der Halter im Hauptteil eingeklebt sein.

Bei der erfindungsgemäßen Trokarvorrichtung kann das Dichtungselement in axialer Richtung über den Halter vorstehen, wobei der vorstehende Teil des Dichtungselementes zum ersten Ende hin weist. Die Trokarvorrichtung kann so ausgebildet sein, daß das Dichtungselement, wenn das Instrument (z.B. ein Endoskop) bestimmungsgemäß in die Durchgangsöffnung eingesetzt ist, in axialer Richtung zusammengedrückt ist, so daß eine Rückstellkraft auf das Instrument wirkt, die zum ersten Ende des Hauptteils hin gerichtet ist.

Ferner kann die Trokarvorrichtung eine Fixiereinheit aufweisen, die das Instrument, wenn es bestimmungsgemäß in die Trokarvorrichtung eingesetzt ist, gegen eine Bewegung hin zum ersten Ende des Hauptteils arretiert. Die Fixiereinheit kann dazu einen quer zur Längsrichtung des Schaftrohres bewegbaren Schieber aufweisen, der in einer ersten Stellung die gewünschte Arretierung bewirkt. Der Schieber kann durch eine Feder in der ersten Stellung gehalten sein.

Wenn der Schieber aus einer ersten Stellung in eine zweite Stellung bewegt wird, bewirkt er keine Arretierung des Instrumentes mehr, so daß dieses aus der Trokarvorrichtung entnommen werden kann. Wenn in der bestimmungsgemäßen Stellung des Instrumentes das Dichtungselement das Instrument mit einer Kraft hin zum ersten Ende des Hauptkörpers beaufschlagt, wird dann dadurch das Instrument in Richtung zum ersten Ende des Hauptteils hin bewegt. Dies erleichtert das Herausnehmen des Instrumentes aus der Trokarvorrichtung.

Das Dichtungselement kann aus einem Elastomer (beispielsweise Silikon) hergestellt sein.

Das Hauptteil, das Schaftrohr, und/oder der Halter können aus Metall und/oder Kunststoff hergestellt sein.

Bei der erfindungsgemäßen Trokarvorrichtung handelt es sich bei dem eingeklemmten Abschnitt der hohlzylinderförmigen Wandung insbesondere um einen sich in Längsrichtung der hohlzylinderförmigen Wandung erstreckenden Abschnitt. Es wird somit nur ein Teil der hohlzylinderförmigen Wandung (in Längsrichtung gesehen) mittels des Halters eingeklemmt.

Bei der erfindungsgemäßen Trokarvorrichtung ist die Ausdehnung des elastischen Dichtelementes in Längsrichtung der hohlzylinderförmigen Wandung bevorzugt größer als die lichte Weite der hohlzylinderförmigen Wandung. Wenn das elastische Dichtungselement beispielsweise einen kreisförmigen Innenquerschnitt aufweist, ist die lichte Weite der Durchmesser dieses Innenquerschnittes. Ansonsten wird darunter insbesondere die maximale Ausdehnung des Innenquerschnittes verstanden.

Die hohlzylinderförmige Wandung weist bevorzugt eine konstante Dicke bzw. Wandstärke auf (ohne Berücksichtigung der durch den Halter bedingten Klemmung).

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Schnittdarstellung einer Ausführungsform der erfindungsgemäßen Trokarvorrichtung, und
- Fig. 2: eine vergrößerte Schnittdarstellung der Dichtungseinheit 8 im Hauptkörper 2 gemäß Fig. 1.

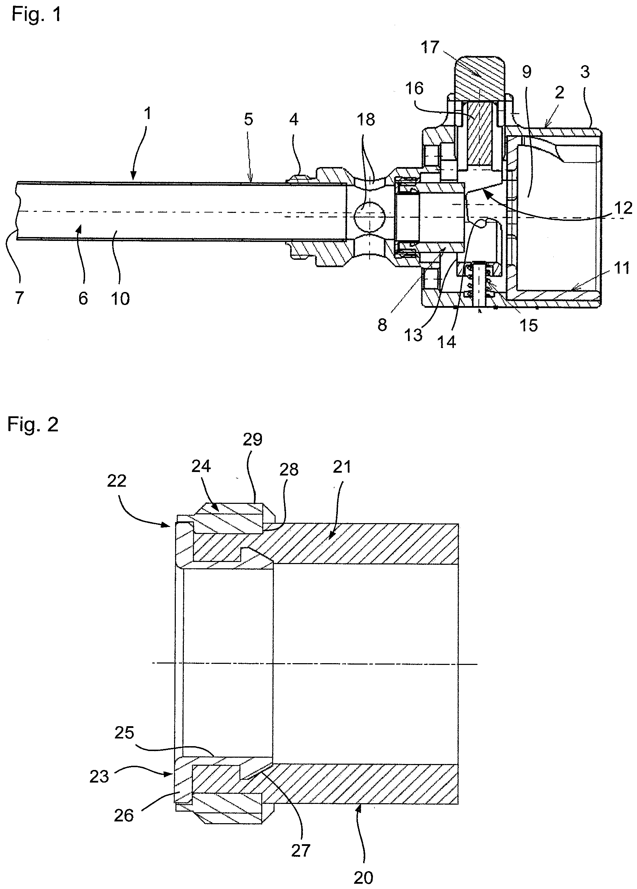

Bei der in Fig. 1 gezeigten Ausführungsform umfaßt die erfindungsgemäße Trokarvorrichtung 1 einen Hauptteil 2 mit einem ersten und einem zweiten Ende 3, 4, ein Schaftrohr 5, das so mit dem zweiten Ende 4 des Hauptteils 2 verbunden ist, daß eine Durchgangsöffnung 6 vorliegt, die sich vom ersten Ende 3 des Hauptteiles 2 bis zum dem Hauptteil 2 abgewandten Ende 7 (nachfolgend auch Schaftrohrende 7 genannt) erstreckt.

In dem Hauptteil 2 ist eine Dichtungseinheit 8 angeordnet, die die Durchgangsöffnung in einen ersten Bereich 9, der von der Dichtungseinheit 8 bis zum ersten Ende 3 des Hauptteiles 2 verläuft, und in einen zweiten Bereich 10, der von der Dichtungseinheit 8 bis zum Schaftrohrende 7 verläuft, aufteilt.

Die Trokarvorrichtung 1 ist ausgebildet, um ein (nicht gezeigtes) Endoskop aufzunehmen, das vom ersten Ende 3 in die Durchgangsöffnung 6 eingeführt wird. Dazu ist im Bereich des ersten Endes 3 im Hauptteil 2 ein Führungsteil 11 angeordnet. Dem Führungsteil 11 ist ferner eine Fixiereinheit 12 nachgeordnet, die ein quer zur Längsrichtung (in Fig. 1 von oben nach unten) bewegbares Fixierelement 13 mit einer Schulter 14 aufweist. Zwischen der Fixiereinheit 12 und dem Hauptteil 2 ist eine Feder 15 vorgesehen, die die Fixiereinheit 12 nach oben drückt und in der in Fig. 1 gezeigten Stellung hält. Das der Feder 15 abgewandte Ende 16 der Fixiereinheit 12 ist aus dem Hauptteil 2 herausgeführt und mit einer Kappe 17 versehen, so daß durch Drücken auf die Kappe 17 in Richtung der Feder 15 die Fixiereinheit 12 gegen die Federkraft nach unten geschoben werden kann.

Das Hauptteil 2 weist (links) neben der Dichtungseinheit 8 Öffnungen 18 auf, die in den zweiten Bereich 10 münden. Über diese Öffnungen 18 kann z.B. eine Spülflüssigkeit in den zweiten Bereich 10 der Durchgangsöffnung 6 eingebracht werden. Die Dichtungseinheit 8 dient dann, wenn das (nicht gezeigte) Endoskop in die Durchgangsöffnung 6 eingesetzt ist, dazu, den ersten Bereich 9 vom zweiten Bereich 10 abzudichten, so daß keine Spülflüssigkeit oder sonstige Flüssigkeit aus dem zweiten Bereich 10 in den ersten Bereich 8 gelangen und das Endoskop verschmutzen kann.

Wenn das Endoskop in die Durchgangsöffnung 6 eingesetzt wird, wird es über das erste Ende 3 in Richtung des Schaftrohres 5 in die Durchgangsöffnung 6 geschoben. Dabei weist das Endoskop eine Ausnehmung auf, die so ausgebildet und angeordnet ist, daß beim Einschieben des Endoskopes der Bereich vor der Ausnehmung gegen die Schulter 14 drückt und somit die Fixiereinheit 12 in Richtung zur Feder 15 hin verschiebt und bei einem weiteren Einführen des Endoskopes aufgrund der Feder 15 die Fixiereinheit 12 in der entgegengesetzten Richtung bewegt wird und dadurch die Schulter 14 in die Ausnehmung einsteht, so daß das Endoskop gegen ein Herausziehen (nach rechts in Fig. 1) aus der Durchgangsöffnung 6 gesichert ist.

Die Dichtungseinheit 8 ist in Fig. 2 vergrößert dargestellt. Sie umfaßt ein elastisches Dichtungselement 20, das aus einem Elastomer (wie z.B. Silikon) gebildet sein kann und das eine hohlzylinderförmige Wandung 21 aufweist. Die Innenkontur der Wandung 21 ist an das einzuführende Endoskop angepaßt. So kann die Kontur kreisförmig, oval, polygonförmig oder eine sonstige Form aufweisen. Das elastische Dichtungselement 20 ist somit als Hohlzylinder ausgebildet. Bevorzugt weist das elastische Dichtungselement 20 ausschließlich die Form eines Hohlzylinders auf.

Ferner umfaßt die Dichtungseinheit 8 einen Halter 22 mit einem Innenteil 23 und einem Außenteil 24. Das Innenteil 23 ist in Form eines Anschlußstutzens ausgebildet und weist einen hohlzylinderförmigen Bereich 25, der an der Innenseite der Wandung 21 anliegt, sowie einen sich an dem linken Ende des hohlzylinderförmigen Bereiches 25 radial nach außen erstreckenden ringförmigen Anschlagbereich auf, der an der Stirnseite der Wandung anliegt. Am rechten Ende des hohlzylinderförmigen Bereiches 25 ist eine radial nach außen vorstehende Schulter 27 ausgebildet, die die Wandung 21 nach außen drückt.

Das Außenteil 24 ist als Klemmhülse ausgebildet, die an der radialen Endfläche des ringförmigen Anschlagbereiches 26 anliegt und einen sich daran anschließenden Fixierbereich 28 aufweist, dessen Innendurchmesser geringer ist als der Außendurchmesser der Wandung 21. Dadurch wird der zwischen Innen- und Außenteil 23, 24 liegende Abschnitt der Wandung eingeklemmt bzw. zusammengedrückt, so daß dadurch das Dichtungselement 20 im Halter 22 fixiert ist.

Das Außenteil 24 weist ferner ein Außengewinde 29 auf, das in ein entsprechendes Innengewinde im Hauptteil 2 (Fig. 1) eingeschraubt ist, so daß dadurch das Dichtungselement 20 sicher im Hauptteil 2 fixiert ist.

Wie bereits beschrieben wurde, wird das Endoskop im eingesetzten Zustand im Hauptteil durch die Fixiereinheit 12 fixiert. Dabei ist die Trokarvorrichtung 1 so ausgelegt, daß das fixierte Endoskop das Dichtungselement 20 in Längsrichtung etwas zusammendrückt. Das Endoskop wird somit von dem Dichtungselement 20 mit einer Kraft in Richtung zum ersten Ende 3 hin beaufschlagt. Wenn das Endoskop aus der Trokarvorrichtung 1 entfernt werden soll, muß ein Benutzer lediglich die Kappe 17 in Richtung der Feder 15 drücken, so daß die Schulter 14 nicht mehr in Eingriff mit der entsprechenden Ausnehmung in dem Endoskop ist. Aufgrund der von dem Dichtelement 20 vorliegenden Kraftbeaufschlagung wird das Endoskop in Richtung zum ersten Ende 3 hin bewegt, so daß es vom Benutzer dann leicht aus der Trokarvorrichtung 1 entnommen werden kann.

Das Innen- und Außenteil können aus Metall (z.B. Edelstahl) und/oder auch aus Kunststoff hergestellt sein.

Natürlich muß das Außenteil 24 kein Außengewinde aufweisen. Es ist auch möglich, das Außenteil 24 im Hauptteil zu verkleben.

Das Hauptteil kann aus Metall (z.B. Edelstahl) und/oder Kunststoff hergestellt sein.

## Patentansprüche

1. Trokarvorrichtung mit
einem ein erstes und ein zweites Ende (3, 4) aufweisenden Hauptteil,
einem mit dem zweiten Ende (4) des Hauptteils (2) verbundenen Schaftrohr (5),
einer sich vom ersten Ende (3) des Hauptteils (2) bis zum dem Hauptteil (2) abgewandten Ende (7) des Schaftrohrs (5) erstreckenden Durchgangsöffnung (6) zur Aufnahme eines Instrumentes,
und einer in dem Hauptteil (2) angeordnete Dichtungseinheit (8),
die ein elastisches Dichtungselement (20) aufweist,
die die Durchgangsöffnung (6) in einen ersten Bereich (9), der von der Dichtungseinheit (8) bis zum ersten Ende (3) des Hauptkörpers (2) verläuft, und in einen zweiten Bereich (10), der von der Dichtungseinheit (8) bis zum dem Hauptteil abgewandten Ende (7) des Schaftrohres (5) verläuft, aufteilt
und die, wenn das Instrument bestimmungsgemäß in die Durchgangsöffnung (6) eingesetzt ist,
die beiden Bereiche (9, 10) gegeneinander abdichtet,
**dadurch gekennzeichnet, daß**
das Dichtungselement (20) eine hohlzylinderförmige Wandung (21) aufweist, als Hohlzylinder ausgebildet ist und die Dichtungseinheit (8) einen im Hauptteil (2) befestigten Halter (22) umfaßt,
wobei der Halter (22) einen Abschnitt der hohlzylinderförmigen Wandung (21) des Dichtungselementes (20) einklemmt, so daß das Dichtungselement (20) im Halter (22) fixiert ist.

2. Trokarvorrichtung nach Anspruch 1,
**dadurch gekennzeichnet, daß** der Halter (22) ein erstes Halteelement (23), das an der Innenseite der Wandung (21) anliegt, und ein zweites Halteelement (24), das an der Außenseite der Wandung (21) anliegt, aufweist.

3. Trokarvorrichtung nach Anspruch 2,
**dadurch gekennzeichnet, daß** das erste Halteelement (23) einen ersten Klemmabschnitt (27) aufweist, der zum Einklemmen des Wandungsabschnittes radial nach außen vorsteht.

4. Trokarvorrichtung nach Anspruch 2 oder 3,
**dadurch gekennzeichnet, daß** das zweite Halteelement (24) einen zweiten Klemmabschnitt (28) aufweist, der zum Einklemmen des Wandungsabschnittes radial nach innen vorsteht.

5. Trokarvorrichtung nach einem der obigen Ansprüche,
**dadurch gekennzeichnet, daß** der Halter (22) ein Außengewinde (29) aufweist, das zur Befestigung des Halters (22) im Hauptteil (2) in ein Innengewinde im Hauptteil (2) eingeschraubt ist.

6. Trokarvorrichtung nach einem der obigen Ansprüche,
**dadurch gekennzeichnet, daß** das Dichtungselement (20) in axialer Richtung über den Halter (22) vorsteht, wobei der vorstehende Teil des Dichtungselementes zum ersten Ende hin weist.

7. Trokarvorrichtung nach einem der obigen Ansprüche,
**dadurch gekennzeichnet, daß** das Dichtungselement (20) aus einem Elastomer hergestellt ist.

8. Trokarvorrichtung nach einem der obigen Ansprüche,
**dadurch gekennzeichnet, daß** im Hauptteil (2) eine Fixiereinheit (12) angeordnet ist, die das Instrument, wenn es bestimmungsgemäß in der Durchgangsöffnung (6) eingesetzt ist, gegen eine Bewegung zum ersten Ende (3) hin arretiert.

9. Trokarvorrichtung nach einem der obigen Ansprüche,
**dadurch gekennzeichnet, daß** das Dichtungselement (20), wenn das Instrument bestimmungsgemäß in die Durchgangsöffnung (6) eingesetzt ist, in axialer Richtung zusammengedrückt ist, so daß eine Rückstellkraft auf das Instrument wirkt, die zum ersten Ende (3) des Hauptteiles (2) hin gerichtet ist.

10. Trokarvorrichtung nach einem der obigen Ansprüche, **dadurch gekennzeichnet, daß** der eingeklemmte Abschnitt der hohlzylinderförmigen Wandung (21) sich in Längsrichtung der hohlzylinderförmigen Wandung erstreckt.

11. Trokarvorrichtung nach einem der obigen Ansprüche,
**dadurch gekennzeichnet, daß** der mittels des Halters (22) eingeklemmte Abschnitt der hohlzylinderförmigen Wandung (21) des Dichtungselementes (20) sich nicht über die gesamte Ausdehnung der hohlzylinderförmigen Wandung (21) in Längsrichtung der hohlzylinderförmigen Wandung (21) erstreckt.

12. Trokarvorrichtung nach einem der obigen Ansprüche,
**dadurch gekennzeichnet, daß** die Ausdehnung des Dichtungselementes (20) in Längsrichtung der hohlzylinderförmigen Wandung (21) größer ist als die lichte Weite der hohlzylinderförmigen Wandung (21).

## Claims

1. A trocar device, comprising
a main part that has a first and a second end (3, 4),
a shaft tube (5) that is connected to the second end (4) of the main part (2),
a through-opening (6), extending from the first end (3) of the main part (2) as far as the end of the end (7) of the shaft tube (5) that faces away from the main part (2), for receiving an instrument,
and a sealing unit (8), which is disposed in the main part (2),
and which has an elastic sealing element (20),
which divides the through-opening (6) into a first region (9) that runs from the sealing unit (8) as far as the first end (3) of the main body (2), and into a second region (10) that runs from the sealing unit (8) as far as the end (7) of the shaft tube (5) that faces away from the main part,
and which, when the instrument has been properly inserted in the through-opening (6), seals off the two regions (9, 10) from each other,
**characterized in that**
the sealing element (20) comprises a hollow-cylindrical wall (21) being formed as a hollow cylinder and the sealing unit (8) comprises a holder (22), which is fastened in the main part (2),
wherein the holder (22) clamps-in a portion of the hollow-cylindrical wall (21) of the sealing element (20), such that the sealing element (20) is fixed in the holder (22).

2. The trocar device according to claim 1,
**characterized in that** the holder (22) has a first holding element (23), which bears against the inside of the wall (21), and a second holding element (24), which bears against the outside of the wall (21).

3. The trocar device according to claim 2,
**characterized in that** the first holding element (23) has a first clamping portion (27), which projects radially outwards for the purpose of clamping-in the wall portion.

4. The trocar device according to claim 2 or 3,
**characterized in that** the second holding element (24) has a second clamping portion (28), which projects radially inwards for the purpose of clamping-in the wall portion.

5. The trocar device according to any of the above claims,
**characterized in that** the holder (22) has an external thread (29), which is screwed into an internal thread in the main part (2) for the purpose of fastening the holder (22) in the main part (2).

6. The trocar device according to any of the above claims,
**characterized in that** the sealing element (20) projects over the holder (22) in the axial direction, wherein the projecting part of the sealing element faces towards the first end.

7. The trocar device according to any of the above claims,
**characterized in that** the sealing element (20) is made of an elastomer.

8. The trocar device according to any of the above claims,
**characterized in that** disposed in the main part (2) there is a fixing unit (12), which, when the instrument has been properly inserted in the through-opening (6), locks the instrument so as to prevent it from moving towards the first end (3).

9. The trocar device according to any of the above claims,
**characterized in that**, when the instrument has been properly inserted in the through-opening (6), the sealing element (20) is compressed in the axial direction, such that a restoring force, directed towards the first end (3) of the main part (2), acts upon the instrument.

10. The trocar device according to any of the above claims, **characterized in that** the clamped-in portion of the hollow-cylindrical wall (21) extends in the longitudinal direction of the hollow-cylindrical wall.

11. The trocar device according to any of the above claims,
**characterized in that** the portion of the hollow-cylindrical wall (21) of the sealing element (20) that is clamped-in by means of the holder (22) does not extend over the entire extent of the hollow-cylindrical wall (21) in the longitudinal direction of the hollow-cylindrical wall (21).

12. The trocar device according to any of the above claims,
**characterized in that** the extent of the sealing element (20) in the longitudinal direction of the hollow-cylindrical wall (21) is greater than the inside width of the hollow-cylindrical wall (21).

## Revendications

1. Dispositif de trocart avec :
une partie principale comportant une première et une deuxième extrémité (3, 4) ;
une tige de tube (5) reliée à la deuxième extrémité (4) de la partie principale (2) ;
une ouverture traversante (6) s'étendant de la première extrémité (3) de la partie principale (2) jusqu'à l'extrémité (7) de la tige de tube (5) opposée à la partie principale (2) pour recevoir un instrument ; et
une unité d'étanchéité (8) disposée dans la partie principale (2)
ladite unité comportant un élément d'étanchéité (20) élastique ;
ladite unité divisant l'ouverture traversante (6) en une première région (9) s'étendant de l'unité d'étanchéité (8) jusqu'à la première extrémité (3) du corps principal (2) et en une deuxième région (10) s'étendant de l'unité d'étanchéité (8) jusqu'à l'extrémité (7) de la tige de tube (5) opposée à la partie principale ; et
ladite unité étanchéifiant l'une par rapport à l'autre les deux régions (9, 10) lorsque l'instrument est inséré dans l'ouverture traversante (6) dans une utilisation conforme à sa destination ;
**caractérisé en ce que** :
l'élément d'étanchéité (20) comporte une paroi en forme de cylindre creux (21) réalisée sous la forme d'un cylindre creux et l'unité d'étanchéité (8) comprenant un support (22) fixé dans la partie principale (2) ;
le support (22) coinçant une section de la paroi en forme de cylindre creux (21) de l'élément d'étanchéité (20) de sorte que l'élément d'étanchéité (20) soit fixé dans le support (22).

2. Dispositif de trocart selon la revendication 1, **caractérisé en ce que** le support (22) comporte un premier élément d'arrêt (23) butant contre le côté intérieur de la paroi (21) et un deuxième élément d'arrêt (24) butant contre le côté extérieur de la paroi (21).

3. Dispositif de trocart selon la revendication 2, **caractérisé en ce que** le premier élément d'arrêt (23) comporte une première section de serrage (27) saillant vers l'extérieur dans le plan radial pour coincer la section de paroi.

4. Dispositif de trocart selon la revendication 2 ou 3, **caractérisé en ce que** le deuxième élément d'arrêt (24) comporte une deuxième section de serrage (28) saillant vers l'intérieur dans le plan radial pour coincer la section de paroi.

5. Dispositif de trocart selon l'une quelconque des revendications précédentes, **caractérisé en ce que** le support (22) comporte un filet extérieur (29) vissé dans un filet intérieur dans la partie principale (2) pour fixer le support (22) dans la partie principale (2).

6. Dispositif de trocart selon l'une quelconque des revendications précédentes, **caractérisé en ce que** l'élément d'étanchéité (20) ressort au-delà du support (22) dans la direction axiale, la partie saillante de l'élément d'étanchéité étant orientée vers la première extrémité.

7. Dispositif de trocart selon l'une quelconque des revendications précédentes, **caractérisé en ce que** l'élément d'étanchéité (20) est fabriqué à partir d'un élastomère.

8. Dispositif de trocart selon l'une quelconque des revendications précédentes, **caractérisé en ce qu'**une unité de fixation (12) est disposée dans la partie principale (2), cette unité de fixation arrêtant l'instrument à l'encontre d'un mouvement allant vers la première extrémité (3) lorsqu'il est inséré dans l'ouverture traversante (6) dans une utilisation conforme à sa destination.

9. Dispositif de trocart selon l'une quelconque des revendications précédentes, **caractérisé en ce que** l'élément d'étanchéité (20) est resserré dans la direction axiale lorsque l'instrument est inséré dans l'ouverture traversante (6) dans une utilisation conforme à sa destination, de sorte qu'une force de rappel agisse sur l'instrument orienté vers la première extrémité (3) de la partie principale (2).

10. Dispositif de trocart selon l'une quelconque des revendications précédentes, **caractérisé en ce que** la section coincée de la paroi en forme de cylindre creux (21) s'étend dans la direction longitudinale de la paroi en forme de cylindre creux.

11. Dispositif de trocart selon l'une quelconque des revendications précédentes, **caractérisé en ce que** la section coincée à l'aide du support (22) de la paroi en forme de cylindre creux (21) de l'élément d'étanchéité (20) ne s'étend pas sur la totalité de l'extension de la paroi en forme de cylindre creux (21) en direction longitudinale der paroi en forme de cylindre creux (21).

12. Dispositif de trocart selon l'une quelconque des revendications précédentes, **caractérisé en ce que** l'extension de l'élément d'étanchéité (20) est plus importante dans la direction longitudinale de la paroi en forme de cylindre creux (21) que la largeur intérieure de la paroi en forme de cylindre creux (21).
